# EUROPÄISCHE PATENTANMELDUNG

(11) **EP 2 722 015 A1**
(43) Veröffentlichungstag der Anmeldung: **23.04.2014**
(21) Anmeldenummer: 12189297.0
(22) Anmeldetag: 19.10.2012
(51) Int. Cl.: A61B 17/72

(54) **Intramedullärer Marknagel**

(71) Anmelder: Rapido Med GmbH, 34560 Frtizlar (DE)
(72) Erfinder: Gregel, Siegbert, 34549 Edertal (DE); Lahme, Steffen, 34513 Waldeck (DE)
(74) Vertreter: Fuchs

(57) **Zusammenfassung**

Die Erfindung befasst sich mit einem Marknagel 1, insbesondere zur geschlossenen Reposition einer Fraktur eines Röhrenknochens durch Einsetzen in eine Bohrung in dessen Markhöhle, mit einem Nagelkern 2 und mit einem im vorderem Bereich des Marknagels 1 angeordneten Kopfstück 5, an dem der Anfang eines Bandes 3, 4 festgelegt ist, welches um den Nagelkern 2 herum aufgewickelt ist und das an dessen Ende im hinteren Bereich an einer Außenbuchse 8 befestigt ist und welches bei einer Drehung entgegen seiner Aufwickelrichtung die radiale Kontur des Marknagels 1 erweitert, wobei der Nagelkern 2 in einer Innenbuchse 9 angeordnet ist und gegenüber dieser nicht in axialer Richtung verschiebbar ist, wobei die Innenbuchse 9 innerhalb der Außenbuchse 8 rotierbar und in Längsrichtung des Marknagels 1 bewegbar ist. Erfindungsgemäß ist vorgesehen, dass die Innenbuchse 9 einen Außenkonus 10 und die Außenbuchse 8 einen zum Außenkonus 10 koaxial ausgebildeten Innenkonus 11 aufweisen, wobei Außenkonus 10 und Innenkonus 11 sich jeweils in Richtung vom Kopfstück 5 weg verjüngen, sich gegenüber liegen und zumindest abschnittsweise gleich ausgebildet sind, wobei die Flächen von Außenkonus 10 und Innenkonus 11 in einer Verriegelungsposition aneinander gepresst sind und in Entriegelungspositionen kein Kraft- und Formschluss zwischen Außenkonus 10 und Innenkonus 11 gegeben ist.

## Beschreibung

Die Erfindung befasst sich mit einem Marknagel, insbesondere zur geschlossenen Reposition einer Fraktur eines Röhrenknochens durch Einsetzen in eine Bohrung in dessen Markhöhle.

Es ist eine Vorrichtung zur Stabilisierung von Röhrenknochenbrüchen sowie von Gelenken, aus der DE 38 35 682 A1 bekannt, die als Stabilisierungselemente ein- oder mehrgängig ineinander gewickelte Schraubenfedern aufweist, die zum Teil um eine Seele in Form eines elastisch verformbaren Drahtes oder eines Bündels von elastisch verformbaren Drähten herumgewickelt sind. Eine solche Schraubenfeder kann an den Enden mit einer Einrichtung zum Festlegen am Knochen ausgeführt sein. Nachteilig an dieser bekannten Vorrichtung ist die Unbestimmtheit der Lage der Schraubenfedern sowie die Unmöglichkeit einer Rotationsverhinderung wie auch einer Fixierung einzelner Bruchstücke im Bereich einer Fraktur, da sich die Federeigenschaften der Schraubenfedern dort nicht behindern lassen.

Weiterhin ist ein Nagelsystem für die intramedulläre Osteosynthese bei Schaftfrakturen aller Art bekannt, DE 197 31 298 A1 , welches einen rohrförmigen Außenkörper mit Durchbrüchen aufweist, aus denen sich Haken nach außen erstrecken, die durch Betätigung eines Innenkörpers nach außen geschwenkt werden können, wodurch sich die Haken an der Innenwand eines Röhrenknochens anlegen können sollen. Dieses Nagelsystem ist extrem aufwändig und weist sehr viele Bauteile auf, kann aber prinzipbedingt nur Punktkontakte mit der Innenfläche eines Röhrenknochens erzeugen und diese auch nur in einem engen und über die Länge identischen Umfangsbereich.

Einfachere Marknägel zur Verriegelungsnagelung sind bekannt, deren Einbau während eines Eingriffs jedoch nur mittels intraoperativer Röntgendarstellung kontrolliert werden kann, was aufgrund der Dauer nicht nur für einen Patienten, sondern insbesondere für das OP-Personal äußerst belastend ist. Des Weiteren müssen solche einfachen Marknägel mittels Querschrauben im Knochen gesichert werden, wozu insbesondere bei langen Oberschenkelknochen, aber auch bei Oberarm und Unterschenkelknochen aufwändige Zielgeräte erforderlich sind, um solche Querschrauben unter möglichst geringer Verletzung der Haut durch diese hindurch punktgenau setzen zu können. Diese zusätzlich in eine Extremität einzubringenden Operationswunden müssen nach einer Operation aber auch nach einer operativen Entfernung der Querschrauben aufwändig versorgt werden, was zusätzliche Belastungen für einen Patienten mit sich bringt.

Aus der EP 1 827 272 B1 ist ein intramedullärer Marknagel bekannt, der einfach aufgebaut ist, sehr praxisorientiert, leicht, schnell, sicher und bei nur geringem Einsatz von intraoperativer Röntgendarstellung eingesetzt werden kann, ohne dabei Eingriffe in unversehrte Bereiche einer Extremität zu erfordern. Dies wird dadurch erreicht, dass im vorderen Bereich des Marknagels ein Kopfstück angeordnet ist, an dem der Anfang eines Bandes festgelegt ist, welches um den Nagelkern herum aufgewickelt ist und das an dessen Ende im hinteren Bereich an einer Außenbuchse befestigt ist und welches bei einer Drehung entgegen seiner Aufwickelrichtung die radiale Kontur des Marknagels erweitert, wobei der Nagelkern an einer Innenbuchse befestigt ist, sodass die Innenbuchse innerhalb der Außenbuchse rotierbar und in Längsrichtung des Marknagels bewegbar ist. Mit diesem Marknagel kann allerdings kein axialer Druck auf den Frakturspalt bei Querbrüchen ausgeübt werden.

Aufgabe der Erfindung ist es deswegen, einen Marknagel zur Verfügung zu stellen, der auch bei axialem Druck seine vom Operateur eingestellte Spannung und Position nicht ändert.

Diese Aufgabe wird durch einen Marknagel mit den Merkmalen des Patentanspruchs 1 gelöst.

Da erfindungsgemäß im vorderen Bereich der Anfang des Bandes - regelmäßig handelt es sich hierbei um ein äußeres Band - festgelegt ist, welches um den Nagelkern herum aufgewickelt ist und dessen Ende im hinteren Bereich an der Außenbuchse drehverriegelbar festgelegt ist, wobei sich bei einer Drehung entgegen seiner Aufwickelrichtung die radiale Kontur des Marknagels erweitert, wird erreicht, dass sich die Außenseite des Bandes an der Innenseite einer Durchgangsbohrung beziehungsweise unmittelbar an die Innenseite des Markraumes eines Röhrenknochens anlegen kann, beziehungsweise durch ein weiteres Drehen entgegen der Aufwickelrichtung eine radial wirkende Kraft erzeugt werden kann, mit der die Einzelteile eines Knochens optimal ausgerichtet und repositioniert geklemmt werden können, wodurch ein optimales Heilungsergebnis erzielt werden kann. Der technische Aufbau eines solchen Marknagels ist extrem einfach, leicht von jedem Notfallarzt zu verstehen und dadurch auch sehr sicher in der korrekten Anwendung. Auf den Einsatz von distalen Fixierschrauben des Nagels kann hier vollständig verzichtet werden, sodass eine postoperative Schnittwundenversorgung in diesem Bereich nicht mehr erforderlich ist. Durch die Klemmkraft des Bandes wird eine starke seitliche kortikale Abstützung und damit eine optimale Frakturstabilisierung erreicht, was des Weiteren zu einem schnelleren Ablauf der Frakturheilung führt. Frakturheilungsstörungen in Folge mangelhafter Frakturfixation können bei der Verwendung des erfinderischen Marknagels vollständig ausgeschlossen werden. Zur Entfernung des Marknagels wird das Band wieder stärker um den Nagelkern herum aufgewickelt, wodurch sich der Außendurchmesser des Marknagels wieder verringert, sodass er problemlos aus der Bohrung oder der Markhöhle herausgezogen werden kann. Da erfindungsgemäß die Innenbuchse einen Außenkonus und die Außenbuchse einen zum Außenkonus koaxial ausgebildeten Innenkonus aufweisen, wobei Außenkonus und Innenkonus sich jeweils in Richtung vom Kopfstück weg verjüngen, sich gegenüber liegen und zumindest abschnittsweise gleich ausgebildet sind, wobei die Flächen von Außenkonus und Innenkonus in einer Verriegelungsposition aneinander gepresst sind und in Entriegelungspositionen kein Kraft- und Formschluss zwischen Außenkonus und Innenkonus gegeben ist, wird eine ungewollte Lockerung der Fixation vermieden, da dieser die Pressung des Außenkonus in den Innenkonus bei einem Druck auf den Knochen in Längsrichtung des Marknagels noch verstärkt aber keine Bewegung wegen des Kraft- und Formschlusses mehr in diese Richtung möglich ist. Ferner kann durch die Verriegelung eine Kompression auf den Frakturspalt eines Röhrenknochens ausgeübt werden.

Eine vorteilhafte Weiterbildung der Erfindung sieht vor, dass die Innenbuchse am kopfstückfernen Ende des Außenkonus einen daran anschließenden Teil mit Außenzylinder und die Außenbuchse am kopfstückfernen Ende des Innenkonus einen daran anschließenden Teil mit Innenzylinder aufweisen, wobei Außenzylinder und Innenzylinder koaxial und im Wesentlichen spielfrei zueinander angeordnet sind. Dadurch wird eine gute und präzise Führung der Innenbuchse samt Nagelkern innerhalb der

Außenbuchse trotz der teilweisen konischen Ausgestaltung erreicht, ohne dass ein Wackeln der beiden Teile zueinander möglich ist.

Eine weitere vorteilhafte Weiterbildung der Erfindung sieht vor, dass am kopfstückfernen Ende des Nagelkerns ein Endstück mit einem Außengewinde angeordnet ist, wobei zumindest ein Teil des Außengewindes nicht von der Außenbuchse in radialer Richtung überdeckt ist. Dadurch kann zum Abschluss des Marknagels eine Abschlusskappe aufgeschraubt werden, die bei einer entsprechenden Ausgestaltung der einzelnen Teile einen stufenlosen Übergang von der Außenbuchse zur Abschlusskappe ermöglicht. Bevorzugt ist dabei, dass das Endstück ein separates Teil zum Nagelkern ist und gegenüber diesem in seiner Lage fixiert ist.

Eine weitere vorteilhafte Weiterbildung der Erfindung sieht vor, dass der Verschiebeweg zwischen Entriegelungsposition und Verriegelungsposition höchstens 10 mm, bevorzugt höchstens 5 mm, besonders bevorzugt höchstens 3 mm, beträgt. Eine solche Bewegung der Außenbuchse auf das Kopfstück zu reicht aus, um eine Kompression zu erzeugen, durch die eine verbesserte Knochenheilung erzielt werden kann. Eine solche Kompression ist bei bestimmten Patientengruppen - wie Krebspatienten, Tumorpatienten, Drogensüchtigen, Alkoholikern und Patienten, die bestimmte Arzneimittel einnehmen - sehr nützlich.

Eine weitere vorteilhafte Weiterbildung der Erfindung sieht vor, dass an der Innenbuchse zwischen dem Außenkonus und dem Außenzylinder eine umlaufende Nut ausgebildet ist. Damit wird der aus fertigungstechnischen Notwendigkeiten in dem Bereich des Übergangs vom Konus zum Zylinder ausgebildete Grat entfernt.

Eine weitere vorteilhafte Weiterbildung der Erfindung sieht vor, dass am kopfstücknahen Ende des Innenkonus an der Außenbuchse eine erste Anschlagfläche und am kopfstücknahen Ende des Außenkonus an der Innenbuchse eine zweite Anschlagfläche ausgebildet sind, die beide im Wesentlichen senkrecht zur Längsrichtung des Marknagels orientiert sind. Dadurch ist die Innenbuchse mit Nagelkern unverlierbar mit der Außenbuchse verbunden und die beiden Teile können vor und während des Einsetzens des Marknagels nicht zu weit ihre relative Position zueinander verändern.

Eine weitere vorteilhafte Weiterbildung der Erfindung sieht vor, dass die erste Anschlagfläche Teil eines von der Außenbuchse separaten Führungsteils ist, wobei das Führungsteil fest mit der Außenbuchse verbunden ist. Dadurch kann bei der Herstellung des Marknagels durch einfache Art und Weise die nötige Montage von Innenbuchse zu Außenbuchse an einer günstigen Stelle vorgenommen werden. Aufgrund der Ausrichtung der beiden Konen ist es nicht möglich, die Innenbuchse von der kopfstückfernen Seite in die Außenbuchse einzuführen. Die feste Verbindung des Führungsteils mit der Außenbuchse erfolgt bevorzugt durch Verschweißen, ist aber auch durch alle anderen, dem Fachmann bekannten Verbindungsmethoden möglich.

Eine weitere vorteilhafte Weiterbildung der Erfindung sieht vor, dass der Nagelkern gerade ausgebildet und mit der Innenbuchse fest verbunden ist. Hierbei handelt es sich um eine einfache und sichere Möglichkeit, diese beiden Teile miteinander zu verbinden. Der Nagelkern muss dabei gerade ausgebildet sein, da das Kopfstück ansonsten bei der Drehung der Innenbuchse samt Nagelkern eine Kreisbewegung vollführen würde, was in einem Knochen nicht möglich ist.

Eine weitere vorteilhafte Weiterbildung der Erfindung sieht vor, dass die Innenbuchse und/oder der Nagelkern hohl ausgebildet sind. Dadurch wird Masse und somit Gewicht eingespart, was aufgrund des reduzierten Materialbedarfs zu einer Preisreduzierung führt und trotzdem eine ausreichende Stabilität des Marknagels gegeben ist.

Eine weitere vorteilhafte Weiterbildung der Erfindung sieht vor, dass die Innenfläche des Führungsteils der Außenbuchse zylindrisch ist und die darin angeordneten Teile der Außenfläche der Innenbuchse oder des Nagelkerns im Wesentlichen dazu spielfrei ausgebildet sind. Dadurch wird zusätzlich oder alternativ zu der Ausgestaltung mit den oben schon angegebenen Innen- und Außenzylinder am kopfstückfernen Ende der Außen- und der Innenbuchse eine gute und präzise Führung der Innenbuchse samt Nagelkern innerhalb der Außenbuchse trotz der teilweisen konischen Ausgestaltung erreicht, ohne dass ein Wackeln der Teile zueinander möglich ist.

Als besonders vorteilhaft ist eine Ausführung hervorzuheben, bei der im vorderen Bereich des Marknagels der Anfang eines weiteren Bandes - regelmäßig eines inneren Bandes - festgelegt ist, welches unter dem ersten Band - regelmäßig einem äußeren Band - um den Nagelkern herum aufgewickelt ist und dessen Ende im hinteren Bereich des Marknagels drehverriegelbar um die Innenbuchse festgelegt ist. Ein solches (inneres) Band vergrößert bei einer Drehung entgegen seiner Aufwicklungsrichtung ebenfalls den Außendurchmesser des Marknagels beziehungsweise legt sich mit seiner Außenseite an der Innenseite des ersten (äußeren) Bandes an und stabilisiert so das Band und damit den gesamten Marknagel. Diese Konstruktion ermöglicht die Verwirklichung einer hohen Steifigkeit, die etwa der eines dünnen Rohres entsprechen kann.

Gemäß weiterer vorteilhafter Ausführungsformen der Erfindung können auch mehrere innere oder äußere Bänder gleich- oder gegensinnig sowie ein- oder mehrgängig um den Nagelkern herum aufgewickelt sein, wobei bei einer einlagigen Version der inneren Bänder diese unmittelbar um den Nagelkern herum aufgewickelt sind und bei einer einlagigen Ausführungsform der äußeren Bänder diese um die inneren Bänder herum aufgewickelt sind. Dieser Variationsreichtum erlaubt es, für die häufigsten Arten von Frakturen von Knochen beliebiger Größe jeweils einen optimal angepassten Marknagel zur Verfügung stellen zu können, da die geforderte Festigkeit quasi beliebig hoch eingestellt werden kann.

Besonders vorteilhaft ist es, wenn die radialen Außenflächen des inneren Bandes und die radialen Innenflächen des äußeren Bandes als gegenseitige Funktionsflächen rau ausgebildet sind, sodass die gemeinsamen Kontaktflächen der Bänder Stabilisierungspunkte eines in eine Durchgangsbohrung eines Knochens eingesetzten und verspannten Marknagels in Form einer kraft- und reibschlüssigen Verbindung erzeugen. Hierdurch wird die Stabilität des Marknagels weiter vergrößert.

Bei einer weiteren vorteilhaften Ausgestaltung des Gegenstandes der Erfindung ist der Nagelkern über seine gesamte Länge hohl ausgebildet und von einer Hülse und/oder einer Welle durchtreten, sodass am vorderen Bereich des Kopfstückes des Marknagels der Anfang eines inneren und/oder äußeren Bandes festgelegt werden kann, wobei vom hinteren Ende des Marknagels dieses über einen entsprechend ausgebildeten Verriegelungsmechanismus von hinten gegen seine Drehrichtung abgewickelt und aufgespannt werden kann. Bei einer praxisorientierten Ausführungsform kann dabei eine deutlich höhere Anzahl von Windungen und hier nur ein einzelnes, von der als Kopfstück ausgebildeten Spitze aus gegensinnig aufgewickeltes Band, dessen innere Wicklungen das innere Band bilden und die äußeren Wicklungen das äußere Band, verwendet werden. Der gegenständliche Nagelkern ist hier bis auf einen Teil im Verriegelungsmechanismus vollständig reduziert, beziehungsweise wird durch einen freien Luftraum ersetzt. Eine solche Version ermöglicht ein nach außen kräftefreies Verspannen des Marknagels, wie bei allen anderen Versionen auch, ohne dass mechanische Rückstellkräfte von einem Knochen aufgenommen oder in diesen eingeleitet werden müssten.

Eine erfinderische Weiterbildung des Marknagels besitzt etwa zwei äußere Bänder von denen eines vom vorderen Bereich des Marknagels aus und eines vom hinteren Bereich des Marknagels aus aufdrehbar sind, sodass eine optimale Anpassung des jeweiligen Bandes an den sich von einer Markraumtaille beidseitig trompetenhaft erweiternden Markraum und damit eine optimale seitliche kortikale Abstützung möglich wird.

Der Marknagel kann je nach Bedarf gerade oder gebogen ausgebildet oder auch entsprechend der geforderten Raumform plastisch verbiegbar sein.

Weiterhin kann der Nagelkern eines Marknagels in vorteilhafter Weise mehrteilig ausgebildet sein, sodass sich diese Teile gegeneinander verdrehen lassen. Dies erweitert die Möglichkeiten zur konstruktiven Gestaltung sehr wesentlich.

Bei einer weiteren sehr vorteilhaften Ausführungsform der Erfindung sind zwei gegeneinander aufgewickelte Bänder an ihren vorderen Enden miteinander verbunden oder aber ein Band von vorne herein einteilig ausgebildet und von seinem vorderen Ende aus gegensinnig um den Nagelkern aufgewickelt, sodass der Nagelkern verkürzt ausgebildet sein kann und sich nur bis zu einem Teilbereich in Richtung des vorderen Endes des Marknagels erstreckt. Der Marknagel kann bei einer solchen Ausgestaltung dünner sein als bei durchgehendem Nagelkern. Bei einer extremen Ausführungsform dieser Ausgestaltung ist der Nagelkern sogar bis auf den Verriegelungsmechanismus reduziert, wodurch quasi im Einbauzustand ein stabiles starres Netzgitter als Marknagel übrig bleibt, was zu einer enormen Materialeinsparung führt. Eine solche Ausführungsform ist besonders bei kleinen Röhrenknochen - wie beispielsweise Ulna und Radius - geeignet.

Eine weitere vorteilhafte Weiterbildung der Erfindung sieht vor, dass der Marknagel aus Stahl besteht. Aufgrund der geringeren Biokompatibilität von Stahl gegenüber Titan wird das Einwachsen auf ein Minimum reduziert. Stahl ist nicht thrombogen, toxisch, allergen, fibrogen oder karzinogen. Es erfolgt kein Abbau zellulärer Elemente, keine Veränderung an Plasmaproteinen und Enzymen und auch keine Gewebsnekrose. Stahl ist korrosionsfest, da keine lonisierung metallischer Atome erfolgt, degradationsfest und auslaugungsfest bei Kontakt mit Blut oder Körperflüssigkeit. Es erfolgt kein Einwachsen osteoblastenähnlicher Knochenzellen durch die mechanische Strukturierung der Implantatoberfläche und die physikalisch-chemischen Eigenschaften des Oberflächenwerkstoffs können eingestellt werden, sodass eine Einkapselung des Implantats anstatt eines Einwachsens erfolgt und eine einfache Metallentfernung gewährleistet ist.

Erfindungsgemäß ist auch die Verwendung des hierin beschriebenen Marknagels zur Ausübung eines Kompressionsdrucks auf einen Frakturspalt in einem Röhrenknochen.

Eine vorteilhafte Ausgestaltungen der Erfindung sowie weitere Vorteile werden darüber hinaus anhand der Zeichnung erläutert. Im Einzelnen zeigen:
- Figur 1: eine teilgeschnittene Ansicht eines erfindungsgemäßen Marknagels mit Abschlusskappe,
- Figur 2: eine geschnittene Teilansicht, insbesondere eines Verriegelungsmechanismus im entriegelten Zustand und
- Figur 3: einen Schnitt gemäß Figur 2 mit dem Verriegelungsmechanismus im verriegelten Zustand.

Der Marknagel 1 besteht aus einem Nagelkern 2, um den von seinem Kopfstück 5 ein inneres Band 4 und ein äußeres Band 3 gegensinnig zueinander aufgewickelt sind, wobei die vorderen Enden der Bänder 3, 4 etwa über ein Laser-Schweißverfahren am Kopfstück 5 ortsfest angeordnet sind. Der Nagelkern 2 weist an seinem kopfstücknahen Ende einen geringeren Durchmesser auf und ist dort in das Kopfstück 5 eingeschoben und mit diesem fest verbunden - beispielsweise durch Verschweißen. Im hinteren Bereich des Marknagels 1 sind die Enden der Bänder 3, 4 an einer Außenbuchse 8 beziehungsweise einer Innenbuchse 9 des Verriegelungsmechanismus 7 angeordnet und dort beispielsweise ebenfalls mittels eines Lasers verschweißt. Die gegensinnig aufgewickelten Bänder 3, 4 überdecken sich an Kontaktflächen, wobei die zueinander gerichteten Oberflächen der Bänder 3, 4 so ausgebildet sind, dass dort eine reibschlüssige Fixierung erfolgt, die den gesamten Marknagel 1 stabilisiert. Dadurch ergibt sich eine verbesserte Fixierung einer Fraktur.

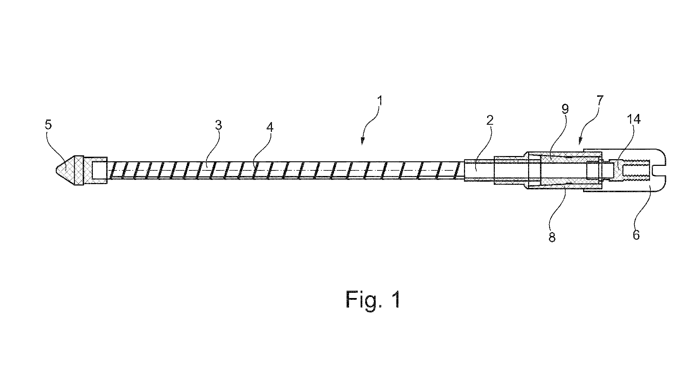

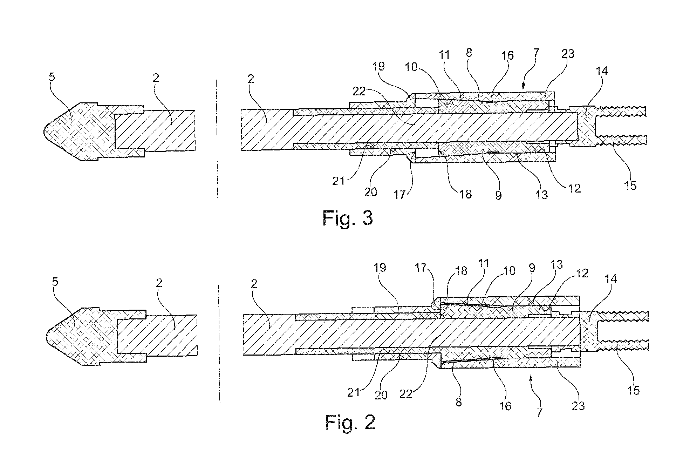

Die in Figur 1 beispielhaft dargestellte Ausführungsform des äußeren Bandes 3 weist Windungen auf, die im Bereich des Kopfstücks 5 eine geringere Breite aufweisen als im Bereich der Außenbuchse 8. Zur dadurch erzielten Wirkung folgen unten weitergehende Ausführungen. Ein Endstück 14 ist mit dem kopfstückfernen Ende des Nagelkerns 2 fest verbunden, beispielsweise durch Verschweißen. Auf dieses wird im verriegelten Zustand, der in Figur 1 dargestellt ist und in Figur 3 noch näher erläutert wird, eine Abschlusskappe 6 geschraubt, deren Funktion weiter unten noch näher erläutert wird.

In den Figuren 2 und 3 sind Längsschnitte durch den Marknagel 1 der Figur 1 dargestellt, wobei die Bänder 3, 4 aus Gründen der Übersichtlichkeit nicht dargestellt sind.

Die erfindungsgemäße Neuerung des Marknagels 1 ist in seinem Verriegelungsmechanismus 7 zu sehen.

Die mit dem äußeren Band 3 (siehe Figur 1) verbundene Außenbuchse 8 ist zweiteilig ausgebildet. Über eine Schweißnaht 22 - andere Verbindungsmethoden sind ebenfalls möglich - ist ein Führungsteil 19 (in den Figuren 2 und 3 ist dies der linke Teil der Außenbuchse 8) mit einem Hauptteil 23 verbunden. Der Hauptteil 23 weist ausgehend von der Schweißnaht 22 nach rechts eine innere Oberfläche in der Form eines Innenkonus 11 auf, der sich nach rechts verjüngt. Direkt im Anschluss an diesen Innenkonus 11 schließt sich einstückig (die beiden Bereiche können auch als separate, fest miteinander verbundene Teile ausgeführt sein) ein Bereich an, dessen Oberfläche die Form eines Innenzylinders 13 aufweist. Der Durchmesser des Innenzylinders 13 ist gleich groß wie der (kleinste) Durchmesser des Innenkonus 11 an seinem rechten Ende. Innenzylinder 13 und Innenkonus 11 sind koaxial ausgerichtet entlang der Mittelachse des Nagelkerns 2.

Der Führungsteil 19 ist in seinem schweißnahtfernen Bereich als Zylinder ausgebildet, der koaxial zum Innenzylinder 13 und Innenkonus 11 angeordnet ist, dessen Innenfläche 20 einen Durchmesser hat, der geringer ist als der an die Schweißnaht anschließende (größte) Durchmesser des Innenkonus 11. Im Bereich der Schweißnaht 22 weist der Führungsteil 19 deshalb eine Schulter auf, die senkrecht auf die Längsachse des Nagelkerns 2 steht und über die er mit dem Hauptteil 23 verschweißt ist. Dadurch wird im Bereich der Schulter eine erste Anschlagfläche 17 gebildet, die sich senkrecht zur Längsachse des Nagelkerns 2 erstreckt.

Die Innenbuchse 7 ist verschiebbar entlang der Längsachse des Nagelkerns 2 innerhalb der Außenbuchse 8 angeordnet. Sie ist zwar zum Nagelkern 2 um dessen Längsachse - einem Gleitlager vergleichbar - rotierbar, aber nicht entlang dieser Achse zu ihm verschiebbar. Dies wird dadurch erreicht, dass der Durchmesser des Nagelkerns 2 am kopfstückfernen Ende geringer ist als in seinem Mittelabschnitt, wodurch ein Anschlag gebildet wird, über den der Nagelkern 2 am kopfstücknahen Ende der Innenbuchse 9 anschlägt; gleichzeitig wird am kopfstückfernen Ende der Innenbuchse 9 durch das fest mit dem Nagelkern 2 verbundenen Endstück 14 ein weiterer Anschlag für den Nagelkern 2 gegenüber der Innenbuchse 9 ausgebildet.

Sie weist im Wesentliche drei Abschnitte auf, die für ihre Funktionalität im Zusammenspiel mit der Außenbuchse 8 von Bedeutung sind: im linken, kopfstücknahen Bereich eine zylindrische Außenfläche 21, der daran anschließende Bereich weist die Form eines Außenkonus 10 auf, an den sich zum kopfstückfernen Ende der Innenbuchse 9 hin ein Bereich in der Form eines Außenzylinders 12 anschließt.

Die zylindrische Außenfläche 21 ist so dimensioniert, dass sie gerade spielfrei in der zylindrischen Innenfläche 20 des Führungsteils 19 der Außenbuchse 8 läuft.

Der Außenzylinder 12 ist so dimensioniert, dass er gerade spielfrei im Innenzylinder des Hauptteils 23 der Außenbuchse 8 läuft.

Der dazwischenliegende Bereich des Außenkonus 10 ist in Richtung der Längsachse des Nagelkerns 2 kürzer ausgebildet als der Innenkonus 11 der Außenbuchse 8. Im Übergangsbereich zwischen Außenkonus 10 und Außenzylinder 12 ist eine umlaufende Nut 16 ausgebildet. Am Übergang des Außenkonus 10 in die Nut 16 ist der Durchmesser des Außenkonus (sein kleinster) geringfügig größer als derjenige des Innenkonus 11 der Außenbuchse 8 an seinem Übergang zum Innenzylinder 13. Durch die Ausführung der Nut 16 in diesem Übergangsbereich wird der aus fertigungstechnischen Notwendigkeiten dort ausgebildete Grat entfernt. Der Öffnungswinkel des Außenkonus 10 ist gleich wie derjenige des Innenkonus 11 der Außenbuchse 8. Im Bereich des Übergangs von der Außenfläche 21 zum Außenkonus 10 weist die Innenbuchse 9 eine senkrecht zur Längsachse des Nagelkerns 2 stehende zweite Anschlagfläche 18 auf, die kleiner ist als die erste Anschlagfläche 17 am kopfstückfernen Ende des Führungsteils 19 der Außenbuchse 8.

Am kopfstückfernen Ende der Innenbuchse 9 ist der Nagelkern 2 mit einem Endstück 14 mittels einer geeigneten, dem Fachmann bekannten Verbindungsmethode fest miteinander verbunden. Das Endstück 14 weist an seinem kopfstückfernen Ende ein Außengewinde 15 auf.

Außenzylinder 12, Außenkonus 10 und Außenfläche 21 der Innenbuchse 9 sind koaxial zueinander und auch koaxial zu den entsprechenden Teilen der Außenbuchse 8 angeordnet. Somit kann sich die Innenbuchse 9 über eine gewisse Länge innerhalb der Außenbuchse 8 bewegen. Die beiden Extrempunkte dieser Bewegungsmöglichkeit sind in den Figuren 2 und 3 dargestellt.

Figur 2 stellt den Marknagel 1 in seinem entriegelten Zustand dar, wie er vor und während des Einführens in den Knochen vorliegt. Hierbei ist die Innenbuchse 9 so in der Außenbuchse 8 angeordnet, dass ihre zweite Anschlagfläche 18 an der ersten Anschlagfläche 17 der Außenbuchse 8 anschlägt. Hierbei berührt der Außenkonus 10 der Innenbuchse 9 den Innenkonus 11 der Außenbuchse 8 nicht. Die Innenbuchse 9 kann somit frei - abgesehen von den Bändern 3, 4, die dies erschweren - innerhalb der Außenbuchse 8 um die Längsachse des Nagelkerns 2 rotiert werden.

Nach dem Einführen des Marknagels 1 in den Knochen und erreichen der gewünschten Position wird zur Fixierung des Marknagels 1 ein Aufdrehen durchgeführt. Hierzu wird die Innenbuchse 9 in der Außenbuchse 8 um die Längsachse des Nagelkerns 2 rotiert, sodass eine Aufweitung der Bänder 3, 4 erfolgt.

Die Bänder 3, 4 (siehe Figur 1) bestehen bevorzugt aus federelastischem Material, etwa aus chirurgischem Stahl, Titan oder körperfreundlichem Kunststoff. Dadurch lassen sich die gewünschten Eigenschaften des Marknagels 1 hinsichtlich der erforderlichen Kontaktkraft wie auch die Art und der Ablauf der Verformung, vorteilhafterweise zunächst von dem vorderen Bereich des Marknagels 1 über die Markraumtaille bis zum sich wieder erweiternden Ende eines Markraumes optimal festlegen. Um eine Aufweitung zu erreichen, bei der sich das äußere Band 3 an die Innenkonturen des Markraumes eines Röhrenknochens anlegt, sind das innere Band 4 und/oder das äußere Band 3 in unterschiedlichen Bereichen des Nagelkerns 2 unterschiedlich breit oder dick ausgebildet (siehe Figur 1) oder aber das innere Band 4 und/oder das äußere Band 3 sind in unterschiedlichen Bereichen des Nagelkerns 2 unterschiedlich stark elastisch vorgespannt. Denkbar ist es auch, dass beispielsweise das äußere Band 3 über seine gesamten Länge oder zumindest teilweise, vom Kopfstück 5 aus gesehen etwa über die ersten 2/3 seiner Länge einen sich kontinuierlich verändernden Querschnitt, insbesondere eine zunehmende Breite aufweist (siehe Figur 1), um die geforderte Funktion zu erfüllen. Eine solche vorteilhafte Konstruktion ermöglicht es auch, bei nicht vorgespannten Bändern 3, 4, dass sich das äußere Band 3 beim Aufdrehen gegen den Drehsinn zunächst im Bereich des Kopfstückes 5 des Marknagels 1 an die Innenkontur eines Knochens anlegt und sich erst beim weiteren Aufdrehen zunächst im mittleren Bereich der Markraumtaille eines Röhrenknochens und anschließend im Endbereich des Marknagels 1 an den sich wieder erweiternden Bereich des Markraumes anlegt.

Ist die gewünschte Aufdrehung der Bänder 3, 4 erreicht, erfolgt eine Überführung des Marknagels 1 in seinen verriegelten Zustand, wie er in Figur 3 dargestellt ist. Hierzu wird die Außenhülse 8 mit einem geeigneten Montagewerkzeug in Richtung auf das Kopfstück 5 zu bewegt, bis ein Klemmsitz des Außenkonus 10 der Innenbuchse 9 am Innenkonus 11 der Außenbuchse 8 gegeben ist. Dies wird schon nach einem Verschiebeweg von 3 mm erreicht. In diesem verriegelten Zustand wird eine weitere Rotation der Innenbuchse 9 in der Außenbuchse 8 ebenso wie eine axiale Verschiebung der Teile zueinander unterbunden. Der Marknagel 1 ist unverrückbar im Knochen fixiert.

Um eine ungewollte Lösung des Klemmsitzes, beispielsweise bei Erschütterungen, zu unterbinden, wird nach Erreichen der in Figur 3 gezeigten Position auf das Außengewinde 15 des Endstücks 14 eine mit einem geeigneten Gewinde versehene Abschlusskappe 6 (siehe Figur 1) als Sicherungsmutter aufgeschraubt, bis diese mit ihrem linken Rand an dem rechten, kopfstückfernen Rand des Hauptteils 23 der Außenbuchse 8 anschlägt. Dann ist gewährleistet, dass auch bei Erschütterungen keine Lageveränderung der Innenbuchse 9 gegenüber der Außenbuchse 8 erfolgen kann.

Damit wird ein Marknagel 1 zur Verfügung gestellt, der trotz geringer Anzahl an benötigten Einzelteilen einen Verriegelungsmechanismus 7 aufweist, der auch bei axialem Druck seine vom Operateur eingestellte Spannung und Position nicht ändert.

### Bezugszeichenliste

- 1: Marknagel
- 2: Nagelkern
- 3: Äußeres Band
- 4: Inneres Band
- 5: Kopfstück
- 6: Abschlusskappe
- 7: Verriegelungsmechanismus
- 8: Außenbuchse
- 9: Innenbuchse
- 10: Außenkonus
- 11: Innenkonus
- 12: Außenzylinder
- 13: Innenzylinder
- 14: Endstück
- 15: Außengewinde
- 16: Nut
- 17: Erste Anschlagfläche
- 18: Zweite Anschlagfläche
- 19: Führungsteil
- 20: Innenfläche
- 21: Außenfläche
- 22: Schweißnaht
- 23: Hauptteil

## Patentansprüche

1. Marknagel (1), insbesondere zur geschlossenen Reposition einer Fraktur eines Röhrenknochens durch Einsetzen in eine Bohrung in dessen Markhöhle,
mit einem Nagelkern (2)
und mit einem im vorderem Bereich des Marknagels (1) angeordneten Kopfstück (5), an dem der Anfang eines Bandes (3, 4) festgelegt ist, welches um den Nagelkern (2) herum aufgewickelt ist und das an dessen Ende im hinteren Bereich an einer Außenbuchse (8) befestigt ist und welches bei einer Drehung entgegen seiner Aufwickelrichtung die radiale Kontur des Marknagels (1) erweitert,
wobei der Nagelkern (2) in einer Innenbuchse (9) angeordnet ist und gegenüber dieser nicht in axialer Richtung verschiebbar ist,
wobei die Innenbuchse (9) innerhalb der Außenbuchse (8) rotierbar und in Längsrichtung des Marknagels (1) bewegbar ist,
**dadurch gekennzeichnet, dass**
die Innenbuchse (9) einen Außenkonus (10) und die Außenbuchse (8) einen zum Außenkonus (10) koaxial ausgebildeten Innenkonus (11) aufweisen,
wobei Außenkonus (10) und Innenkonus (11) sich jeweils in Richtung vom Kopfstück (5) weg verjüngen, sich gegenüber liegen und zumindest abschnittsweise gleich ausgebildet sind,
wobei die Flächen von Außenkonus (10) und Innenkonus (11) in einer Verriegelungsposition aneinander gepresst sind und in Entriegelungspositionen kein Kraft- und Formschluss zwischen Außenkonus (10) und Innenkonus (11) gegeben ist.

2. Marknagel (1) nach Patentanspruch 1, wobei die Innenbuchse (9) am kopfstückfernen Ende des Außenkonus (10) einen daran anschließenden Teil mit Außenzylinder (12) und die Außenbuchse (8) am kopfstückfernen Ende des Innenkonus (11) einen daran anschließenden Teil mit Innenzylinder (13) aufweisen, wobei Außenzylinder (12) und Innenzylinder (13) koaxial und im Wesentlichen spielfrei zueinander angeordnet sind.

3. Marknagel (1) nach einem der vorstehenden Patentansprüche, wobei am kopfstückfernen Ende des Nagelkerns (2) ein Endstück (14) mit einem Außengewinde (15) angeordnet ist, wobei zumindest ein Teil des Außengewindes (15) nicht von der Außenbuchse (8) in radialer Richtung überdeckt ist.

4. Marknagel (1) nach einem der vorstehenden Patentansprüche, wobei der Verschiebeweg zwischen Entriegelungsposition und Verriegelungsposition höchstens 10 mm, bevorzugt höchstens 5 mm, besonders bevorzugt höchstens 3 mm, beträgt.

5. Marknagel (1) nach einem der Patentansprüche 2 bis 4, wobei an der Innenbuchse (9) zwischen dem Außenkonus (10) und dem Außenzylinder (12) eine umlaufende Nut (16) ausgebildet ist.

6. Marknagel (1) nach einem der vorstehenden Patentansprüche, wobei am kopfteilnahen Ende des Innenkonus (11) an der Außenbuchse (8) eine erste Anschlagfläche (17) und am kopfteilnahen Ende des Außenkonus (10) an der Innenbuchse (9) eine zweite Anschlagfläche (18) ausgebildet sind, die beide im wesentlichen senkrecht zur Längsrichtung des Marknagels (1) orientiert sind.

7. Marknagel (1) nach Patentanspruch 6, wobei die erste Anschlagfläche (17) Teil eines von der Außenbuchse (8) separaten Führungsteils (19) ist, wobei das Führungsteil (19) fest mit der Außenbuchse (8) verbunden ist.

8. Marknagel (1) nach einem der vorstehenden Patentansprüche, wobei der Nagelkern (2) gerade ausgebildet und mit der Innenbuchse (9) fest verbunden ist.

9. Marknagel (1) nach einem der vorstehenden Patentansprüche, wobei die Innenbuchse (9) und/oder der Nagelkern (2) hohl ausgebildet sind.

10. Marknagel (1) nach einem der Patentansprüche 7 bis 9, wobei die Innenfläche (20) des Führungsteils (19) der Außenbuchse (8) zylindrisch ist und die darin angeordneten Teile der Außenfläche (21) der Innenbuchse (9) oder des Nagelkerns (2) im wesentlichen dazu spielfrei ausgebildet sind.

11. Marknagel (1) nach einem der vorstehenden Patentansprüche, wobei er aus Stahl besteht.
